# EUROPEAN PATENT SPECIFICATION

(11) **EP 2 863 819 B1**
(45) Date of publication and mention of the grant of the patent: **09.08.2017**
(21) Application number: 13731182.5
(22) Date of filing: 14.05.2013
(51) Int. Cl.: A61B 17/88, A61B 17/80

(54) **SOFT TISSUE BONE REDUCTION FORCEPS**
WEICHGEWEBE-KNOCHENREDUKTIONSZANGE
PINCE DE RÉDUCTION OSSEUSE UTILISABLE SUR TISSUS MOUS

(30) Priority: 20.06.2012 US 201261662049 P
(43) Date of publication of application: 29.04.2015
(73) Proprietor: Synthes GmbH, 4436 Oberdorf (CH)
(72) Inventor: DELL'OCA, Alberto A. Fernandez, Montevideo 11500 (UY); HATT, Alexander, CH-4528 Zuchwill (CH)
(74) Representative: Klunker IP Patentanwälte PartG mbB
(86) International application number: PCT/US2013/040865
(87) International publication number: WO 2013/191819

(56) References cited:
- US-A1- 2005 049 629
- US-A1- 2010 152 789

## Description

### Background Information

Clamps are commonly used in bone fixation procedures to correct the alignment of two or more bone fragments and to hold the fragments in the corrected alignment until a permanent fixation device can be applied to the bone. Typical bone clamps require the use of a surgical approach to permit gripping ends of the clamps to come into direct contact with the target portions of the bone to apply a clamping force thereto. These bone clamps are generally inserted to the fracture site through a large incision formed adjacent to the fracture site or through multiple openings formed at a plurality of predetermined positions adjacent to the target region. Such bone clamps generally comprise towel-clip gripping end portions having sharpened opposing points to facilitate non-slip grasping of the bone.

Bone clamps with two pivotable arms are disclosed in US 2010/0152789 A1 and US 2005/0049629 A1. At least one of the arms has a spherical tip at its distal end that pivotally connects to an insert. The insert is configured for skin contact.

### Summary of the Invention

The present invention is directed to a system for reducing a bone fracture as described in the claims. The device according to the invention comprises a first arm extending from a handle at a proximal end to a distal end having a first substantially spherical tip, a second arm extending from a handle at a proximal end to a distal end having a second substantially spherical tip, the second arm being pivotally connected to the first arm and an insert removably attachable to a selected one of the first and second tips, the insert comprising a connection mechanism configured to lockingly engage the selected one of the first and second spherical tips, the insert including a first face sized and shaped to be seated over a target portion of skin in an operative configuration, the first face having a first substantially planar portion and a second substantially curved portion attached thereto, the first and second portions defining a contour conforming to a contour of the target portion of skin.

### Brief Description of the Drawings

Fig. 1 shows a perspective view of an exemplary device according to the invention with an insert attached thereto;
Fig. 2 shows a perspective view of the device of Fig. 1 with the insert separated therefrom;
Fig. 3 shows a first partial cross-sectional view of the insert for the device of Fig. 1;
Fig. 4 shows a first perspective view of the insert for the device of Fig. 1;
Fig. 5 shows a second perspective view of the insert for the device of Fig. 1;
Fig. 6 shows a cross-section of the first arm of the device taken at the line A-A;
Fig. 7 shows a cross-section of the first arm of the device taken at the line B-B;
Fig. 8 shows a cross-section of the first arm according to an alternate embodiment of the invention;
Fig. 9 shows a cross-section of the first arm according to another alternate embodiment of the invention.

### Detailed Description

The present invention may be further understood with reference to the following description and the appended drawings, wherein like elements are referred to with the same reference numerals. The exemplary embodiments of the present invention relate to a system for the reduction of fractured, shattered or otherwise damaged bones using a bone clamp. The exemplary bone clamp of the present invention may be used for the fixation of any fracture and comprises a pair of forceps arms having distal ends configured to aid in reduction of the bone. The first and second arms of the exemplary bone forceps extend to a rounded or otherwise blunted distal end. A removable insert attached to the distal end of the second arm adapts the second end to apply manipulative force to the bone indirectly - e.g., through the skin - while the distal end of the first arm is generally placed in indirect contact with the bone. For example, the distal end of the first arm may contact a bone plate or an implant positioned over the bone, as will be described in greater detail later on. The first and second arms of one exemplary bone forceps according to the invention are shaped to permit reduction of fractures of larger bones. Specifically, a distal portion of the first arm curves radially outward with a radius of curvature selected to prevent unwanted contact with an outer surface of a bone over which the first arm is positioned - i.e., the curvature is selected to prevent contact between the bone and any part of the first arm other than the distal end thereof. A distal portion of the second arm extends along a path selected so that, when the insert coupled to the distal end thereof is in contact with a target portion of skin adjacent to the bone to be treated, the rest of the second arm does not contact the skin. In accordance with an exemplary embodiment of the invention, the distal portion of the second arm may be flattened to facilitate the seating of the forceps on an operating table, as will be described in greater detail below.

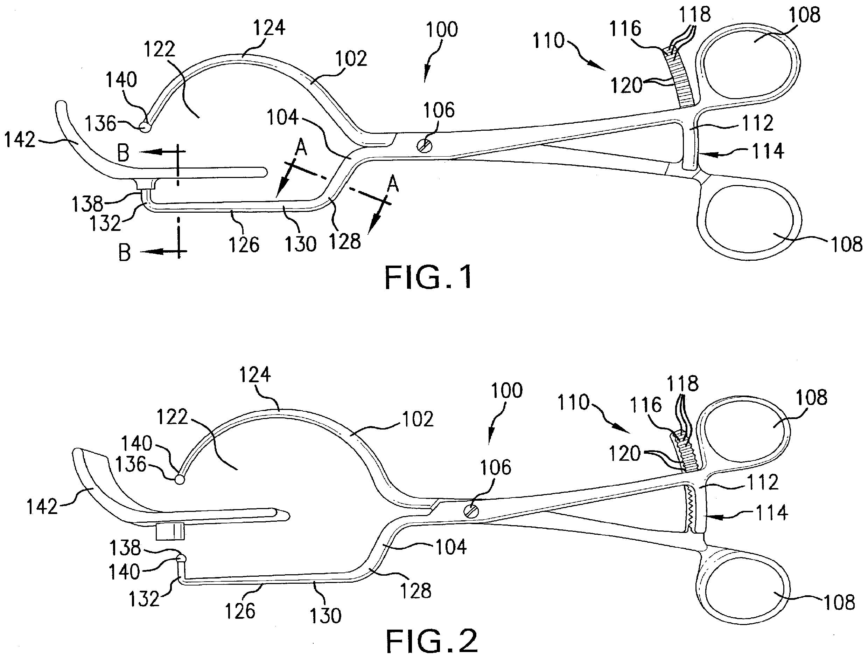

As shown in Figs. 1 - 5, a bone clamping device 100 according to a first exemplary embodiment of the present invention comprises first and second arms 102, 104 pivotally joined to one another at a pivot point 106 including a screw, pivot pin or other mechanism known in the art. Proximal ends of the first and second arms 102, 104 comprise finger loops 108 to permit gripping and actuation of the first and second arms 102, 104, as those skilled in the art will understand. The first and second arms 102, 104 also comprise a ratcheting locking mechanism 110 adjacent to the finger loops 108. Specifically, a first elongated member 112 on the first arm 102 includes a tabbed protrusion 114 extending toward a second elongated member 116 extending from the second arm 104. A length of the first elongated member 112 according to this embodiment is shorter than that found in conventional ratcheting mechanisms. The second elongated member 116 includes a plurality of raised tabs 118 facing the tabbed protrusion 114 to engage the tabbed protrusion 114 in the same manner as in known forceps ratchet locking mechanisms. As those skilled in the art will understand, the first and second elongated members 112, 116 extend along an arc substantially centered at the pivot point 106. A length of the second elongated member 118 is selected so that, as first and second distal ends 136, 138 are drawn together, tabbed protrusion 114 comes into contact with the tabs 118, locking the first and second arms 102, 104 in a ratchet-like manner. As those skilled in the arms will understand, a recess 120 is defined between each of the tabs 118, the recess 120 seating the tabbed protrusion 114 therein. A first face of each of the tabs 118 facing the recess 120 is angled to permit the second arm 104 to slide thereover into the following recess 120 while a second face of each tab 118 facing away from the second arm 104 extends substantially orthogonally out from the tab 118 to lock the second arm 104 and prevent it from slipping thereover away from the first arm 102. This temporarily locks positions of the first and second aims 102, 104 relative to one another, as those skilled in the art will understand. In an exemplary embodiment of the invention, the locking mechanism 110 employs soft-ratcheting, permitting an operator of the bone clamping device 100 to manipulate the locking mechanism 110 with one hand, as those skilled in the art will understand.

Each of the first and second arms 102, 104 extends substantially in a plane from the finger loop 108 at its proximal end to the pivot point 106. The portions of the first and second arms 102, 104 extending distally from the pivot point 106 initially curve radially outward away from one another and then curve back toward one another so that, when the first and second distal ends 136, 138 are brought into contact with one another, a space 122 is defined therebetween. Specifically, the first arm 102 includes a first bow 124 having a first radius of curvature selected to prevent direct contact thereof with any portion of a patient's anatomy when the distal end 136 contacts a first target portion of the anatomy. In an exemplary embodiment, the first target portion of the anatomy is a target location on a bone having a fracture to be reduced and the radius of curvature of the first bow 124 is greater than a radius of curvature of a portion of soft tissue covering the bone (not shown). In an exemplary embodiment, the first bow 124 is substantially hemispherical. It is noted that the hemispherical shape of the first bow 124 is exemplary only and that any other shape may be used without deviating from the scope of the invention. The second arm 104 extends along a second path 126 a portion of which is rectangular. Specifically, the second path 126 includes first wall 128 extending radially away from the first arm 102 at a first predetermined angle and leading to a second wall 130 extending substantially parallel to a longitudinal axis of the bone clamping device 100. The second wall 130 leads to a third wall 132 extending substantially orthogonal thereto and having a length selected such that the space 122 has a predetermined size corresponding to a thickness of the tissue between the first and second distal ends 136, 138, respectively, when the first distal end 136 engages the first target portion of the anatomy and the second distal end 138 engages a second target portion of the anatomy opposite the first target portion. An outer portion of the second wall 130 is configured and dimensioned to permit the bone clamping device 100 to rest stably on an operating table, as those skilled in the art will understand.

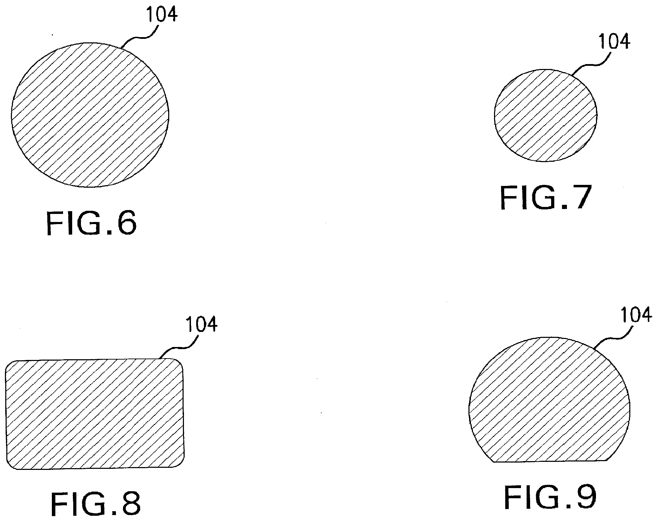

Substantially tapered transitions are provided between the first and second and second and third walls 128, 130, 132 such that there are no sharpened edges on the second arm 104. It is noted that the depicted shape of the second bow 126 is exemplary only and that any other shape may be used without deviating from the scope of the invention. In an exemplary embodiment, both the first and second bows 124, 126 are formed by manipulating a substantially cylindrical rod to form the shapes depicted in Figs. 1 and 2. In a first exemplary embodiment, each of the first and second arms 102, 104 may have a substantially circular cross-section as shown in Figs. 6 and 7. In another embodiment, one or both of the first and second bows 124, 126 may have a non-circular cross-section. For example, the cross-section may be a substantially rounded rectangle as shown in Fig. 8. In another embodiment, the cross-section may be formed by a circle having an elongated flat cutout extending therethrough as shown in Fig. 9. In this embodiment, the bone clamping device 100 may rest on the elongated flat cutout on the operating table. A cross-sectional diameter of the first and second bows 124, 126 may decrease in a distal direction such that a diameter of each of the first and second bows 124, 126 taken at a proximal portion at line A-A is greater than a diameter taken at a distal portion at line B-B, as shown in Figs. 6 and 7. As those skilled in the art will understand, this configuration reduces a profile of a distal portion of the bone clamping device 100 to aid in handling and insertion thereof to a target location on/in the body.

First and second distal ends 136, 138, respectively, of the first and second arms 102, 104 each include blunt (e.g., substantially spherical) tips 140. In an exemplary embodiment, the first and second arms 102, 104 and tips 140 are both formed of a suitable biocompatible material (e.g., stainless steel) as would be understood by those skilled in the art. As those skilled in the art will understand, the blunt tips 140 permit the application of a constrictive force to soft tissue adjacent to a target bone while minimizing trauma thereto. Conventional bone clamps adapted to be employed in direct contact with bone employ sharpened distal tips to aid in fracture reduction. These conventional forceps require either a large incision or multiple incisions to permit the sharpened tips to directly contact the bone. In contrast, the blunt tips 140 of this embodiment of the invention permit the clamping of the bone with or without an incision on a dorsal side of the bone, as will be described in greater detail with respect to the exemplary method below. In an exemplary embodiment, the diameters of the blunt tips 140 are substantially equivalent to one another and selected to permit the mounting of a plastic insert 142 thereover. In a preferred embodiment, a diameter of the blunt tip 140 may be approximately 4 mm, although any other dimension may be used without deviating from the scope of the invention. A diameter of the rod forming the bows 124, 126 may be approximately 6 mm at the line A-A, decreasing to approximately 2.5 mm at the line B-B, although any other dimensions may be used without deviating from the scope of the invention.

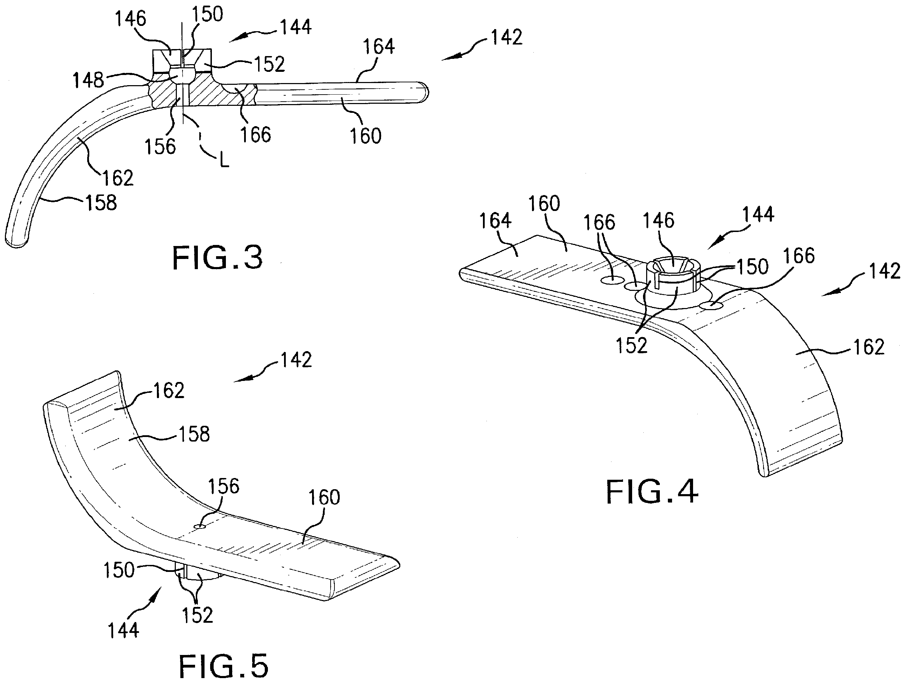

As shown in greater detail in Figs. 3 - 5, the exemplary insert 142 according to the invention includes a connection mechanism 144 permitting removable attachment of the insert 142 to the blunt tip 140 any plurality of times as needed. The insert 142 may be formed of a biocompatible plastic material (e.g., PPSG LSG, etc.). The connection mechanism 144 includes a first substantially conical channel 146 extending thereinto and opening into a cavity 148 sized and shaped to compressively receive the blunt tip 140. As those skilled in the art will understand, the shape of the cavity 148 is preferably selected to permit the blunt tip 150 to rotate when positioned therewithin, permitting pivotal movement of the insert 142 relative to the second arm 104. The pivotal movement allows the insert 142 to adjust to the contour of a portion of soft tissue over which a skin-contacting surface 158 of the insert 142 is positioned to increase an area of contact therewith to prevent slipping. In an exemplary embodiment, outer walls of the conical channel 146 may be angled at approximately 30° relative to a longitudinal axis L of the channel 146, although any other angle may be used without deviating from the scope of the invention to permit pivotal movement of the insert 142 within a desired range. A diameter of the conical channel 146 tapers down in size from an outer opening toward the cavity 148. The connection mechanism 144 further includes a plurality of elongated slots 150 extending through an outer wall of the conical channel 146, the elongated slots 150 defining a plurality of tabs 152 which may deflect radially outward to permit pivotal movement of the insert 142 relative to the second arm 104 without fracturing. That is, the tabs 152 are sufficiently flexible to deflect radially outward as the insert 142 is pivoted relative to the third wall 132 outside of the ±30° range dictated by a shape of the conical channel 146. In another embodiment, the deflection may be limited to approximately ±15° relative to the longitudinal axis L. As those skilled in the art will understand, the tabs 152 prevent a fracture of the connection mechanism 144 when the insert 142 is inadvertently pivoted outside of a desired range. Furthermore, the tabs 152 are biased to the position shown in Figs. 3 - 4 so that they grip the blunt tip 140 and retain it within the cavity 148. The cavity 148 may further open to an elongated, substantially cylindrical channel 156 extending to the skin-contacting wall 158 of the insert 142, the channel 156 being dimensioned to facilitate cleaning of the cavity 148.

The insert 142 is formed with a substantially arced "J" shape including a first substantially planar portion 160 and a second curved portion 162 having a radius of curvature selected conforming to a radius of curvature of a portion of soft tissue covering a bone over which the insert 142 is placed.

As shown in Figs. 3 - 4, an outer wall 164 of the insert 142 located opposite the skin-contacting wall 158 may include one ore more undercuts 166 adjacent the connection mechanism 144. The undercuts 166 are provided as an alternate engagement mechanism for the blunt tip 140 to be used in lieu of the connection mechanism 144. Specifically, the connection mechanism 144 is positioned along the insert 142 to permit optimal frictional contact with a bone having a diameter within a predetermined range. When a diameter of the bone is greater or smaller than the predetermined range, a user may manually position the insert 142 over the skin and position the blunt tip 140 in a desired undercut 166 to permit frictional clamping of the insert 142 over the bone. Specifically, in larger bones, the blunt tip 140 of the second arm 104 may engage an undercut 166 on the planar portion 160. In smaller bones, the blunt tip 140 of the second arm 104 may engage the undercut 166 provided on the curved portion 162. Each of the undercuts 166 has a shape corresponding to the spherical shape of the blunt tip 140 to prevent disengagement of the blunt tip 140 therefrom in an operative configuration. Specifically, the blunt tip 140 may be held over the selected undercut 166 by a frictional pressure applied by the bone clamping device 100 when positioned over the bone, as described in greater detail earlier.

The outer wall 164 may further include a label (not shown) provided anywhere thereon to indicate specific details about the insert including, but not limited to product type, lot number, article number, country of origin, company logo, etc. In one embodiment, the label may be etched into the outer wall 164. Dimensions of the insert 142 may be selected to conform to the requirements of a particular procedure. In one exemplary embodiment, the insert 142 may be approximately 20 mm. wide, 65.8 mm. long and have a thickness of approximately 3.5 mm, although any other measurements may be used without deviating from the scope of the invention.

The exemplary system according to the present invention permits the use of the clamping device in minimally invasive bone fixation procedures where, for example, only one incision is to be made adjacent a bone fracture site. In accordance with an exemplary method, a minimally invasive incision is formed through the skin adjacent a site at which a bone fragment is separated from a long bone (e.g., tibia, humerus, femur, ulna, etc.). It is noted that although the method is described with respect to the fixation of fractures of long bone, the exemplary bone clamping device 100 may be used for the fixation of any bone. The bone clamping device 100 is used to reduce a fracture in preparation for permanent fixation via, for example, a bone plate (not shown). The insert 142 is then positioned over the blunt tip 140 of the second arm 104. The blunt tip 140 of the first arm 104 is then inserted through the incision into contact with a bone plate or other bone implant preciously positioned over the bone fragment while the insert 142 is positioned against the skin at a location substantially opposing the location of the bone fragment and the incision. Specifically, the blunt tip 140 of the first arm 102 may engage, for example, a screw hole of the bone plate (not shown) to provisionally lock a position of the first arm 102 relative thereto. Specifically, the insert 142 is separated from the blunt tip 140 of the first arm 102 in the direction of desired movement of the bone fragment so that, as the first and second arms 102, 104 are drawn toward one another, the bone fragment is moved against the long bone to reduce the fracture. As the skin-contacting wall 158 of the insert 142 is placed over the skin, the insert 142 pivots relative to the blunt tip 140 until the skin-contacting wall 158 is firmly seated in a contacting configuration against the skin. This pivotal movement permits the insert 142 to conform the curvature of the skin so that a compressive force applied thereby is evenly distributed over the entire skin-contacting wall 158 to minimize trauma to the skin and underlying tissue as a result of the compressive force. As those skilled in the art will understand, the exemplary insert 142 according to the invention enhances the stability of the bone clamping device 100 as it applies compressive force to the bone by being pressed against the skin adjacent to the bone. The first and second arms 102, 104 are then manipulated until the bone fragments are brought into a desired alignment with one another. As the distal ends of the first and second arms 102, 104 are drawn together, the locking mechanism 110 prevents the first and second arms 102, 104 from being inadvertently drawn apart and allows the user to release the device 100 while maintaining a desired compressive force on the bone fragment and the long bone. When the fracture has been stabilized (e.g., through the insertion of a bone screw or Kirschner wire through the bone plate), the user disengages the tabbed protrusion 114 from the tabs 118 by applying a release force to one or both of the first and second arms 102, 104, the release force directed substantially perpendicular to a plane housing the first and second arms 102, 104.

## Claims

1. A device (100) for reducing a bone fracture, comprising:
a first arm (102) extending from a handle at a proximal end to a distal end (136) having a first substantially spherical tip (140);
a second arm (104) extending from a handle at a proximal end to a distal end (138) having a second substantially spherical tip (140), the second arm (104) being pivotally connected to the first arm (102); and
an insert (142) removably attachable to a selected one of the first and second spherical tips (140), the insert (142) comprising a connection mechanism (144) configured to lockingly engage the selected one of the first and second spherical tips (140), the insert (142) including a first face (158) sized and shaped to be seated over a target portion of skin in an operative configuration,
**characterized in that** the first face (158) has a first substantially planar portion (160) and a second substantially curved portion (162) attached thereto, the first and second portions (160, 162) defining a contour conforming to a contour of the target portion of skin.

2. The device of claim 1, wherein the connection mechanism (144) includes a substantially conical channel (146) opening into a substantially spherical cavity (148) dimensioned to seat the selected one of the first and second spherical tips (140) therewithin while permitting pivotal movement of the insert (142) relative thereto within a desired angular range.

3. The device of claim 2, wherein the desired angular range is defined by a magnitude of an angle enclosed by opposing walls of the conical channel (146).

4. The device of claim 2 or 3, wherein the connection mechanism (144) includes first and second elongated slots (150) extending through an outer wall of the conical channel (146) and defining first and second deflectable tabs (152).

5. The device of any one of claims 2 to 4, further comprising an elongated channel (156) leading from the spherical cavity (148) to the first face (158) of the insert (142) to facilitate cleaning of the spherical cavity (148).

6. The device of any one of claims 1 to 5, wherein the first and second arms (102, 104) include respective first and second bows (124, 126) extending radially outward from a longitudinal axis of the device (100) to prevent interference of the first and second arms (102, 104) with the bone in an operative configuration clamping the bone.

7. The device of any one of claims 1 to 5, wherein a portion of the first arm (102) located distally of a pivot point (106) includes a curved first bow (124) extending away from a longitudinal axis of the device (100) and having a substantially hemispherical shape.

8. The device of any one of claims 1 to 6, wherein a portion of the second arm (104) located distally of a pivot point includes a second bow (126) extending away from the longitudinal axis of the device (100) and having a shape forming a portion of a rectangle.

9. The device of claim 8, wherein the second bow (126) includes a first substantially planar wall (128) extending away from the first arm (102) at a first angle relative to the longitudinal axis, a second substantially planar wall (130) extending parallel to the longitudinal axis and a third substantially planar wall (132) extending toward the first arm (102) at a second angle relative to the longitudinal axis and terminating at the second spherical tip (140).

10. The device of claim 9, wherein the second planar wall (130) includes an elongated flat permitting seating of the second bow (126) thereon.

11. The device of any one of claims 1 to 10, wherein the insert (142) is positioned on the second arm (104).

12. The device of any one of claims 1 to 11, wherein the first spherical tip (140) is substantially the same size as the second spherical tip (140).

13. The device of any one of claims 1 to 12, further comprising a locking mechanism (110), preferably a ratchet mechanism, temporarily locking a position of the first and second arms (102, 104) relative to one another until released.

14. The device of any one of claims 1 to 13, wherein the connection mechanism (144) includes a plurality of undercuts (166) disposed about a second face (164) of the insert (142) opposing the first face (158), each of the undercuts (166) being dimensioned to engage one of the first and second spherical tips (140).

## Patentansprüche

1. Vorrichtung (100) zur Reposition einer Knochenfraktur, umfassend:
einen ersten Arm (102), der sich von einem Griff an einem proximalen Ende zu einem distalen Ende (136), das eine erste im Wesentlichen kugelförmige Spitze (140) aufweist, erstreckt,
einen zweiten Arm (104), der sich von einem Griff an einem proximalen Ende zu einem distalen Ende (138), das eine zweite im Wesentlichen kugelförmige Spitze (140) aufweist, erstreckt, wobei der zweite Arm (104) schwenkbar mit dem ersten Arm (102) verbunden ist, und
einen Einsatz (142), der an einer Ausgewählten der ersten und zweiten kugelförmigen Spitze (140) entfernbar anbringbar ist, wobei der Einsatz (142) einen Verbindungsmechanismus (144) umfasst, der eingerichtet ist, verriegelbar mit der Ausgewählten der ersten und zweiten kugelförmigen Spitze (140) zusammenzuwirken, wobei der Einsatz (142) eine erste Fläche (158) enthält, die bemessen und geformt ist, auf einen Zielteil von Haut in einer operativen Konfiguration aufgesetzt zu werden,
**dadurch gekennzeichnet, dass** die erste Fläche (158) einen ersten im Wesentlichen ebenen Teil (160) und einen daran angebrachten zweiten im Wesentlichen gekrümmten Teil (162) aufweist, wobei der erste und zweite Teil (160, 162) eine Kontur definieren, die einer Kontur des Zielteiles der Haut entspricht.

2. Vorrichtung nach Anspruch 1, wobei der Verbindungsmechanismus (144) einen im Wesentlichen konischen Kanal (146) aufweist, der sich in eine im Wesentlichen kugelförmige Kavität (148) hinein öffnet, die dimensioniert ist, die Ausgewählte der ersten und zweiten kugelförmigen Spitze (140) darin zu lagern, während sie eine Schwenkbewegung des Einsatzes (142) relativ dazu in einem gewünschten Winkelbereich erlaubt.

3. Vorrichtung nach Anspruch 2, wobei der gewünschte Winkelbereich durch einen Betrag eines Winkels definiert ist, der durch gegenüberliegende Wände des konischen Kanals (146) eingeschlossen ist.

4. Vorrichtung nach Anspruch 2 oder 3, wobei der Verbindungsmechanismus (144) einen ersten und zweiten länglichen Schlitz (150) enthält, die sich durch eine äußere Wand des konischen Kanals (146) hindurch erstrecken und eine erste und zweite biegbare Lasche (152) definieren.

5. Vorrichtung nach einem der Ansprüche 2 bis 4, des Weiteren umfassend einen länglichen Kanal (156), der von der kugelförmigen Kavität (148) zur ersten Fläche (158) des Einsatzes (142) führt, um ein Reinigen der kugelförmigen Kavität (148) zu erleichtern.

6. Vorrichtung nach einem der Ansprüche 1 bis 5, wobei der erste und zweite Arm (102, 104) einen entsprechenden ersten und zweiten Bogen (124, 126) enthalten, die sich von einer Längsachse der Vorrichtung (100) radial nach außen erstrecken, um eine Überschneidung des ersten und zweiten Arms (102, 104) mit dem Knochen in einer operativen Konfiguration, in der der Knochen festgeklemmt wird, zu vermeiden.

7. Vorrichtung nach einem der Ansprüche 1 bis 5, wobei ein Teil des ersten Arms (102), der sich distal eines Schwenkpunkts (106) befindet, einen gekrümmten ersten Bogen (124) enthält, der sich weg von einer Längsachse der Vorrichtung (100) erstreckt und eine im Wesentlichen halbkugelförmige Form aufweist.

8. Vorrichtung nach einem der Ansprüche 1 bis 6, wobei ein Teil des zweiten Arms (104), der sich distal eines Schwenkpunkts befindet, einen zweiten Bogen (126) enthält, der sich weg von der Längsachse der Vorrichtung (100) erstreckt und eine Form aufweist, die einen Teil eines Rechtecks bildet.

9. Vorrichtung nach Anspruch 8, wobei der zweite Bogen (126) eine erste im Wesentlichen ebene Wand (128), die sich in einem ersten Winkel relativ zur Längsachse weg von dem ersten Arm (102) erstreckt, eine zweite im Wesentlichen ebene Wand (130), die sich parallel zur Längsachse erstreckt, und eine dritte im Wesentlichen ebene Wand (132), die sich in einem zweiten Winkel relativ zur Längsachse in Richtung des ersten Arms (102) erstreckt und an der zweiten kugelförmigen Spitze (140) endet, enthält.

10. Vorrichtung nach Anspruch 9, wobei die zweite ebene Wand (130) eine längliche Abflachung enthält, die ein Lagern des zweiten Bogens (126) darauf erlaubt.

11. Vorrichtung nach einem der Ansprüche 1 bis 10, wobei der Einsatz (142) auf dem zweiten Arm (104) positioniert ist.

12. Vorrichtung nach einem der Ansprüche 1 bis 11, wobei die erste kugelförmige Spitze (140) im Wesentlichen die gleiche Größe wie die zweite kugelförmige Spitze (140) aufweist.

13. Vorrichtung nach einem der Ansprüche 1 bis 12, des Weiteren umfassend einen Verriegelungsmechanismus (110), vorzugsweise ein Rastmechanismus, der eine Position des ersten und zweiten Arms (102, 104) relativ zueinander temporär verriegelt, bis er gelöst wird.

14. Vorrichtung nach einem der Ansprüche 1 bis 13, wobei der Verbindungsmechanismus (144) eine Mehrzahl von Kuhlen (166) enthält, die auf einer zweiten Fläche (164) des Einsatzes (142) gegenüber der ersten Fläche (158) angeordnet sind, wobei jede der Kuhlen (166) dimensioniert ist, mit einer der ersten und zweiten kugelförmigen Spitze (140) zusammenzuwirken.

## Revendications

1. Dispositif (100) destiné à réduire une fracture osseuse, comprenant :
un premier bras (102) s'étendant d'une poignée sur une extrémité proximale à une extrémité distale (136) présentant un premier bout en grande partie sphérique (140) ;
un second bras (104) s'étendant d'une poignée sur une extrémité proximale à une extrémité distale (138) présentant un second bout en grande partie sphérique (140), le second bras (104) étant raccordé par pivotement au premier bras (102), et
un insert (142) pouvant être attaché de manière amovible sur un bout sélectionné parmi les premier et second bouts sphériques (140), l'insert (142) comprenant un mécanisme de raccordement (144) configuré pour venir en prise par verrouillage avec le un bout sélectionné parmi les premier et second bouts sphériques (140), l'insert (142) incluant une première face (158) présentant une taille et une forme permettant l'assise sur une portion cible de peau dans une configuration opératoire,
**caractérisé en ce que** la première face (158) présente une première portion en grande partie plane (160) et une seconde portion en grande partie incurvée (162) attachée à celle-ci, les première et seconde portions (160, 162) définissant un contour se conformant à un contour de la portion cible de peau.

2. Dispositif selon la revendication 1, dans lequel le mécanisme de raccordement (144) inclut un canal en grande partie conique (146) débouchant dans une cavité en grande partie sphérique (148) dimensionnée pour l'assise du bout sélectionné parmi les premier et second bouts sphériques (140) dans celui-ci tout en permettant un mouvement de pivotement de l'insert (142) par rapport à celui-ci dans les limites d'une plage angulaire souhaitée.

3. Dispositif selon la revendication 2, dans lequel la plage angulaire souhaitée est définie par une grandeur d'un angle enfermé par des parois opposées du canal conique (146).

4. Dispositif selon la revendication 2 ou 3, dans lequel le mécanisme de raccordement (144) inclut des première et seconde fentes allongées (150) s'étendant à travers une paroi extérieure du canal conique (146) et définissant des première et seconde languettes pouvant être déviées (152).

5. Dispositif selon l'une quelconque des revendications 2 à 4, comprenant en outre un canal allongé (156) allant de la cavité sphérique (148) à la première face (158) de l'insert (142) afin de faciliter le nettoyage de la cavité sphérique (148).

6. Dispositif selon l'une quelconque des revendications 1 à 5, dans lequel les premier et second bras (102, 104) incluent des premier et second arcs (124, 126) s'étendant radialement vers l'extérieur à partir d'un axe longitudinal du dispositif (100), afin d'empêcher l'interférence des premier et second bras (102, 104) avec l'os dans une configuration opératoire serrant l'os.

7. Dispositif selon l'une quelconque des revendications 1 à 5, dans lequel une portion du premier bras (102) située de manière distale par rapport à un point de pivotement (106) inclut un premier arc incurvé (124) s'étendant à distance d'un axe longitudinal du dispositif (100) et présentant une forme en grande partie hémisphérique.

8. Dispositif selon l'une quelconque des revendications 1 à 6, dans lequel une portion du second bras (104) située de manière distale par rapport à un point de pivotement inclut un second arc (126) s'étendant à distance de l'axe longitudinal du dispositif (100) et présentant une forme formant une portion d'un rectangle.

9. Dispositif selon la revendication 8, dans lequel le second arc (126) inclut une première paroi en grande partie plane (128) s'étendant à distance du premier bras (102) avec un premier angle par rapport à l'axe longitudinal, une deuxième paroi en grande partie plane (130) s'étendant parallèlement à l'axe longitudinal, et une troisième paroi en grande partie plane (132) s'étendant vers le premier bras (102) avec un second angle par rapport à l'axe longitudinal et terminant sur le second bout sphérique (140).

10. Dispositif selon la revendication 9, dans lequel la deuxième paroi plane (130) inclut une facette allongée permettant l'assise du second arc (126) sur celle-ci.

11. Dispositif selon l'une quelconque des revendications 1 à 10, dans lequel l'insert (142) est positionné sur le second bras (104).

12. Dispositif selon l'une quelconque des revendications 1 à 11, dans lequel le premier bout sphérique (140) est en grande partie de la même taille que le second bout sphérique (140).

13. Dispositif selon l'une quelconque des revendications 1 à 12, comprenant en outre un mécanisme de verrouillage (110), de préférence un mécanisme à cliquet, verrouillant temporairement une position des premier et second bras (102, 104) l'un par rapport à l'autre jusqu'à relâchement.

14. Dispositif selon l'une quelconque des revendications 1 à 13, dans lequel le mécanisme de raccordement (144) inclut une pluralité de contre-dépouilles (166) disposées autour d'une seconde face (164) de l'insert (142) en opposition avec la première face (158), chacune des contre-dépouilles (166) étant dimensionnée pour venir en prise avec un des premier et second bouts sphériques (140).
